(19) Europäisches Patentamt
European Patent Office
Office européen des brevets

(11) Veröffentlichungsnummer: **0 143 888**
**B1**

(12) # EUROPÄISCHE PATENTSCHRIFT

(45) Veröffentlichungstag der Patentschrift: **08.03.89**

(51) Int. Cl.⁴: **C 07 J 53/00**

(21) Anmeldenummer: **84109135.8**

(22) Anmeldetag: **01.08.84**

(54) **Verfahren zur Herstellung von 17-Alpha-acyloxy-6-chlor-1-alpha, 2-alpha-methylen-4,6-pregnadien-3,20-dionen.**

(30) Priorität: **01.09.83 DE 3331824**

(43) Veröffentlichungstag der Anmeldung: **12.06.85 Patentblatt 85/24**

(45) Bekanntmachung des Hinweises auf die Patenterteilung: **08.03.89 Patentblatt 89/10**

(84) Benannte Vertragsstaaten: **AT BE CH DE FR GB IT LI LU NL SE**

(56) Entgegenhaltungen:
**US-A-3 234 093**

**CHEMISCHE BERICHTE, Band 102, Nr. 8, August 1969, Seiten 2570-2582, Weinheim, DE; H. LAURENT et al.: "Darstellung und Reaktionen von 6.7-disubstituierten 3-Oxo-1alpha.2alpha-methylen-delta4-steroiden"**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Junghans, Klaus, Dr., Hanstedter Weg 8, D-1000 Berlin 41 (DE)**

**EP 0 143 888 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Verfahren zur Herstellung von 17α-Acloxy-6-chlor-1α,2-methylen-4,6-pregnadien-3,20-dion.

Die Erfindung betrifft ein Verfahren zur Herstellung von 17α-Acyloxy-6-chlor-1α,2α-methylen-3,20-dionen der allgemeinen Formel I

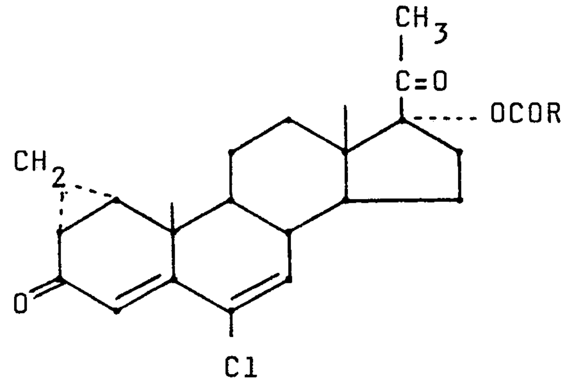

(I),

worin R eine Alkylgruppe mit bis zu 5 Kohlenstoffatomen oder eine Phenylgruppe darstellt, welches dadurch gekennzeichnet ist, daß man eine Verbindung der allgemeinen Formel II

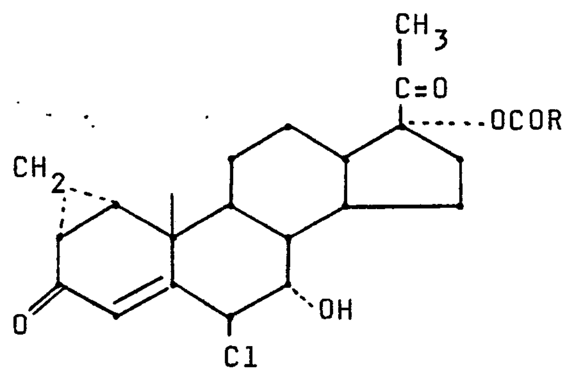

(II),

worin R die obengenannte Bedeutung besitzt, in Gegenwart einer starken Säure mit einer Verbindung der allgemeinen Formel III

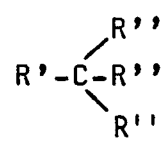

(III),

worin

R' ein Wasserstoffatom oder eine Alkylgruppe mit bis zu 4 Kohlenstoffatomen und R" Alkoxygruppen, Alkylthiogruppen oder Dialkylaminogruppen mit jeweils 1 bis 4 Kohlenstoffatomen im Alkylrest bedeuten, umsetzt.

Verfahren zur Herstellung von 17α-Acyloxy-6-chlor-1α,2α-methylen-4,6-pregnadien-3,20-dionen der allgemeinen Formel I aus Verbindungen der allgemeinen Formel II sind vorbekannt (Chemische Berichte 102, 1969, 2570 - 2582), jedoch sind die nach dem vorbekannten Verfahren erzielten Ausbeuten sehr unbefriedigend.

Es wurde nun gefunden, daß man diese Verbindungen überraschenderweise mit Hilfe des erfindungsgemäßen Verfahrens in wesentlich einfacherer Weise und unter Erzielung signifikant günstigerer Ausbeuten synthetisieren kann.

Die für das erfindungsgemäße Verfahren erforderlichen Verbindungen der allgemeinen Formel III sind vorwiegend Orthoester einer Carbonsäure mit bis zu 4 Kohlenstoffatomen, vorzugsweise der Essigsäure, Orthoester der Ameisensäure, Orthoester der Thioessigsäure oder Dialkylketale des Dimethylformamids. Als Alkoholkomponenten der Orthoester werden vorzugsweise Dimethylester und Diethylester verwendet. Insbesondere werden als Verbindungen der Formel III der Orthoameisensäuretrimethylester, der Orthoameisensäuretriethylester, der Orthoessigsäuretrimethylester, der Orthoessigsäuretriethylester, das Dimethylformamiddimethylketal oder Dimethylformamiddiethylketal verwendet. Diese Substanzen werden entweder als alleinige Lösungsmittel verwendet oder vorzugsweise in Kombination mit weiteren inerten Lösungsmittel in einem Verhältnis von etwa einem mol pro mol Verbindung der Formel II. Geeignete inerte Lösungsmittel sind beispielsweise chlorierte Kohlenwasserstoffe wie Dichlormethan, Trichlormethan,

Tetrachlorethylen oder Trichlorethylen, Ether wie Diethylether, Diisobutylether, Glykoldimethylether, Dioxan oder Tetrahydrofuran, dipolare, aprotische Lösungsmittel, wie Dimethylformamid oder Hexamethylphosphorsäuretriamid oder aromatische, flüssige Kohlenwasserstoffe wie Benzol, Toluol, Xylole etc..

Die Reaktion wird in Gegenwart starker Säuren die man vozugsweise in katalytischen Mengen (etwa 0,01 bis 0,2 mol pro mol Steroid) verwendet, durchgeführt. Geeignete Säuren sind zum Beispiel anorganische Säuren wie Schwefelsäure, wasserfreie Salzsäure und Perchlorsäure, Alkylsulfonsäuren, wie die Methansulfonsäure, Arylsulfonsäuren, wie die p-Toluolsulfonsäure, Trichlormethylessigsäure, Trifluormethylessigsäure oder stark saure Ionenaustauscher wie Amberlite® IR 120. Die Reaktion wird vorzugsweise bei 0° C bis 100° C durchgeführt, am einfachsten erfolgt die Umsetzung bei Raumtemperatur.

Die Ausgangsverbindungen der allgemeinen Formel II können in einfacher Weise mittels verdünnter wässriger Salzsäure aus den entsprechenden 6α,7α-Epoxiden hergestellt werden, wie folgende Darstellungsmethode zeigt:

10,0 g 17α-Acetoxy-6α,7α-epoxy-1αa,2α-methylen-4-pregnen-3,20-dion werden mit 50 ml Tetrahydrofuran und 25 ml verdünnter Salzsäure (8 ml konz. Salzsäure und 17 ml Wasser) versetzt und 15 Minuten lang bei Raumtemperatur gerührt. Dann setzt man der Reaktionsmischung 200 ml Wasser zu, extrahiert mit Dichlormethan, trocknet die organische Phase über Magnesiumsulfat, engt sie im Vakuum ein und erhält 10,8 g 17α-Acet-oxy-6β-chlor-7α-hydroxy-1α,2α-methylen-4-pregnen-3,20-dion vom Schmelzpunkt 154 - 156° C.

Die nachfolgenden Ausführungsbeispiele dienen zur näheren Erläuterung des erfindungsgemäßen Verfahrens.

**Beispiel 1**

5,0 g 17α-Acetoxy-6β-chlor-7α-hydroxy-1α,2α-methylen-4-pre-gnen-3,20-dion werden in 25 ml Dichlormethan mit 0,1 g p-Toluolsulfonsäure und 3,0 ml Orthoameisensäuretrimethylester versetzt. Man läßt die Reaktionsmischung 4 Stunden lang bei 0° C stehen, versetzt sie mit 20 ml Wasser und rührt sie 15 Minuten lang. Dann trennt man die organische Phase ab, trocknet sie über Natriumsulfat und engt sie im Vakuum ein. Man erhält so 4,8 g 17α-Acetoxy-6-chlor-1α,2α-methylen-4,6-pregnadien-3,20-dion vom Schmelzpunkt 202 bis 205° C (Reinheit 80 %-ig).

**Beispiel 2**

5,0 g 17α-Acetoxy-6β-chlor-7α-hydroxy-1α,2α-methylen-4-pre-gnen-3,20-dion werden in 90 ml Toluol mit 0,2 g p-Toluolsulfonsäure und 5,0 ml Orthoessigsäuretriethylester versetzt und 6 Stunden lang bei Raumtemperatur aufbewahrt. Die Reaktionsmischung wird wie im Beispiel 1 beschrieben aufbereitet und man erhält 4,6 g 17α-Acetoxy-6-chlor-1α,2α-methylen-4,6-pregnadien-3,20-dion vom Schmelzpunkt 200 bis 202° C (Reinheit 78 %-ig).

**Beispiel 3**

5,0 g 17α-Acetoxy-6β-chlor-7α-hydroxy-1α,2α-methylen-4-pre-gnen-3,20-dion werden in 60 ml Tetrahydrofuran mit 0,15 ml Methansulfonsäure und 3,0 ml Dimethylformamiddiethylketal versetzt. Man läßt die Reaktionsmischung 3,5 Stunden lang stehen, versetzt sie mit 150 ml Wasser, extrahiert sie mit Dichlormethan, trocknet die organische Phase mit Magnesiumsulfat, engt sie im Vakuum ein und erhält 4,7 g 17α-Acetoxy-6-chlor-1α,2α-methylen-4,6-pregnadien-3,20-dion vom Schmelzpunkt 201 bis 204° C (Reinheit 80 %-ig).

**Patentanspruch**

Verfahren zur Herstellung von 17α-Acyloxy-6-chlor-1α,2α-methylen-3,20-dionen der allgemeinen Formel I

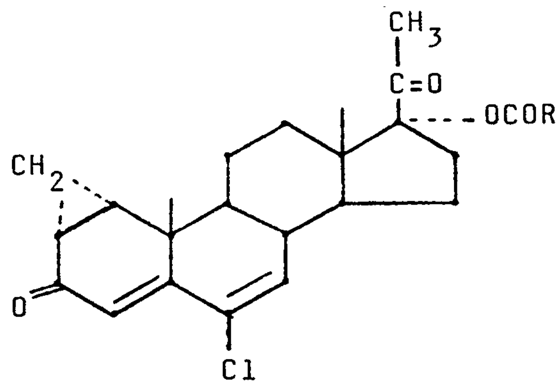

(I),

worin R eine Alkylgruppe mit bis zu 5 Kohlenstoffatomen oder eine Phenylgruppe darstellt, dadurch gekennzeichnet, daß man eine Verbindung der allgemeinen Formel II

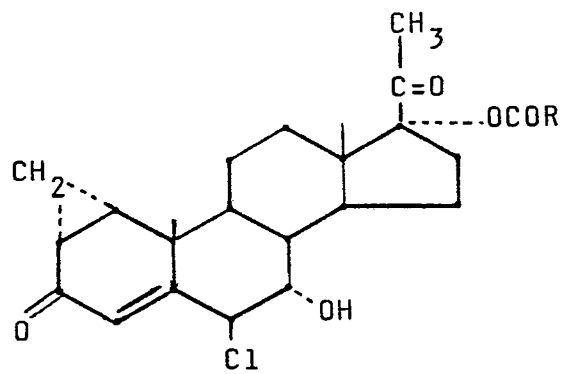

(II),

worin R die obengenannte Bedeutung besitzt, in Gegenwart einer starken Säure mit einer Verbindung der allgemeinen Formel III

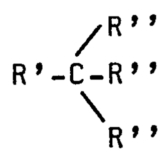

(III),

worin
R' ein Wasserstoffatom oder eine Alkylgruppe mit bis zu 4 Kohlenstoffatomen und R'' Alkoxygruppen, Alkylthiogruppen oder Dialkylaminogruppen mit jeweils 1 bis 4 Kohlenstoffatomen im Alkylrest bedeuten, umsetzt.

**Claim**

Process for the manufacture of 17α-acyloxy-6-chloro-1α,2α-methylene-3,20-diones of the general formula I

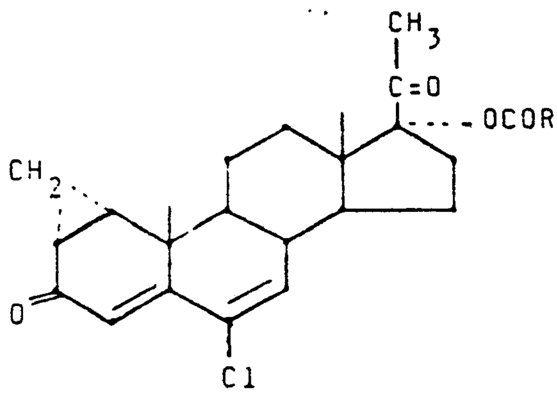

(I),

in which R is an alkyl group having up to 5 carbon atoms, or a phenyl group, characterized in that a compound of the general formula II

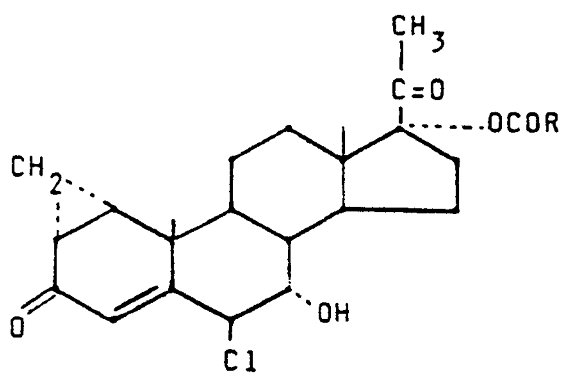

(II),

in which R has the above-mentioned meaning, is reacted in the presence of a strong acid with a compound of the general formula III

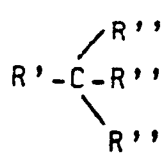

(III)

in which
R' is a hydrogen atom or an alkyl group having up to 4 carbon atoms, and R" represents alkoxy groups, alkylthio groups or dialkylamino groups each having from 1 to 4 carbon atoms in the alkyl radical.

**Revendication**

Procédé de préparation d'acyloxy-17α chloro-6 méthylène-1α,2α diones-3,20 qui répondent à la formule générale I

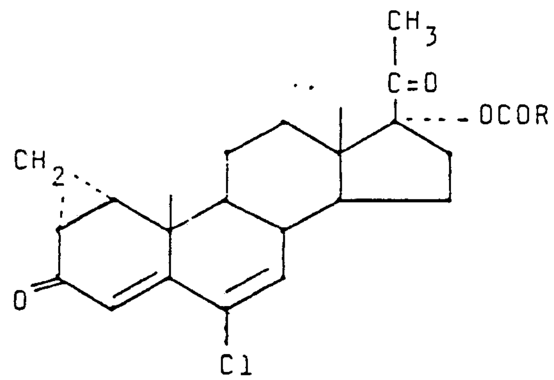

(I)

dans laquelle R représente un radical alkyle contenant au plus 5 atomes de carbone ou représente un radical phényle, procédé caractérisé en ce qu'on fait réagir un composé répondant à la formule générale II

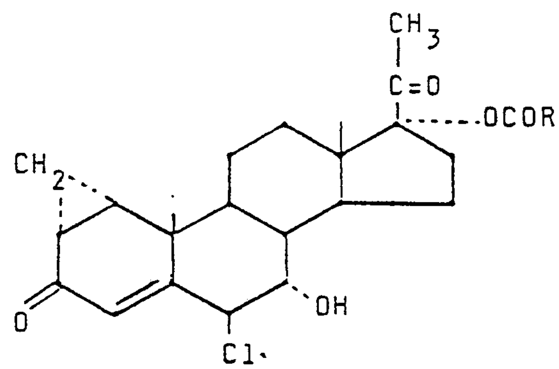

(II)

dans laquelle R a la signification qui lui a été donnée cidessus, en présence d'un acide fort, avec un composé répondant à la formule générale III

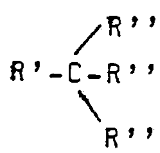

(III)

dans laquelle R représente un atome d hydrogène ou un radical alkyle contenant au plus 4 atomes de carbone et les R" représentent chacun un radical alcoxy, alkylthio ou dialkylamino dont la partie alkyle ou chacune des parties alkyles contient de 1 à 4 atomes de carbone.